# EUROPÄISCHE PATENTSCHRIFT

(11) **EP 3 290 064 B1**
(45) Veröffentlichungstag und Bekanntmachung des Hinweises auf die Patenterteilung: **24.10.2018**
(21) Anmeldenummer: 16187314.6
(22) Anmeldetag: 06.09.2016
(51) Int. Cl.: A61M 1/00, A61M 3/02, A61M 5/152

(54) **SYSTEM ZUR MESSUNG EINER MENGE AN FLÜSSIGKEIT IN EINEM ELASTISCHEN BEUTEL**
SYSTEM FOR MEASURING AN AMOUNT OF LIQUID IN AN ELASTIC BAG
SYSTEME DE MESURE D'UNE QUANTITE D'UN LIQUIDE DANS UN SAC PLASTIQUE

(43) Veröffentlichungstag der Anmeldung: 07.03.2018
(73) Patentinhaber: Fritz Ruck Ophthalmologische Systeme GmbH, 52249 Eschweiler (DE)
(72) Erfinder: Klomp, Manfred, 6336 AM Hulsberg (NL)
(74) Vertreter: Schwarz & Partner Patentanwälte OG

(56) Entgegenhaltungen:
- DE-C1- 3 637 771
- US-A- 5 563 584
- US-A1- 2014 114 236
- US-A1- 2014 276 639

## Beschreibung

Die Erfindung betrifft ein System zur Messung einer Menge an Flüssigkeit in einem elastischen Beutel, wobei der elastische Beutel zusätzlich zur durch die Flüssigkeit gebildeten Flüssigkeitsphase eine Gasphase aufweist und wobei in Betriebslage des elastischen Beutels im unteren Bereich des elastischen Beutels eine Flüssigkeitsöffnung zur Entnahme von Flüssigkeit aus dem elastischen Beutel ausgebildet ist, mit zumindest zwei Drückelementen, welche an gegenüberliegenden Wandungen des elastischen Beutels anliegen und zueinander beabstandet sind, zumindest einer Antriebseinheit, welche zum Antreiben zumindest eines Drückelements ausgebildet ist, wobei die zumindest zwei Drückelemente durch Antreiben zumindest eines Drückelements aufeinander zu bewegbar sind, zumindest einem Sensor, und einer Steuereinheit, welche zum Kommunizieren mit dem zumindest einen Sensor und mit der zumindest einen Antriebseinheit ausgebildet ist.

Die Druckschrift US 6,491,661 B1 offenbart ein Druckregelsystem für ein Infusionsgerät, welches einen elastischen Beutel, zwei Drückelemente in Form von Platten, zwei Federn, ein Ventil, einen Drucksensor und zwei Antriebseinheiten umfasst. Der elastische Beutel ist über einen flexiblen Schlauch angesteckt an eine Flüssigkeitsöffnung des elastischen Beutels mit dem Ventil verbunden. Eine der Platten ist beweglich gelagert und die andere Platte ist mittels nicht näher ausgeführter Zusatzelemente fixiert, wobei die zwei Antriebseinheiten zum Antreiben der beweglich gelagerten Platte ausgebildet sind. Die Platten sind parallel zueinander angeordnet und liegen an gegenüberliegenden Wandungen des elastischen Beutels an, wodurch der elastische Beutel zwischen den Platten eingeklemmt ist. Durch Verkleinerung eines Abstands zwischen den Platten durch Antreiben der beweglich gelagerten Platte mittels der zwei Antriebseinheiten kann ein Druck im Beutel und infolge ein Flüssigkeitsdruck, mit dem Flüssigkeit aus dem elastischen Beutel abgegeben wird, erhöht werden, wobei mittels des Drucksensors und einer integrierten Steuerung ein vorgegebener Druck im elastischen Beutel eingehalten werden kann. Ferner weist das Druckregelsystem einen Volumenstromsensor auf, welcher eine Abgaberate an Flüssigkeit aus dem elastischen Beutel misst, wodurch eine Menge an bereits aus dem elastischen Beutel abgegebener Flüssigkeit errechenbar ist.

Ein ähnliches Druckregelsystem ist auch aus der Druckschrift US 2014/0114236 A1 bekannt.

Als nachteilig bei dem aus der Druckschrift US 6,491,661 B1 und der Druckschrift US 2014/0114236 A1 bekannten Druckregelsystem erweist sich, dass der Volumenstromsensor zum Messen der Abgaberate an Flüssigkeit aus dem elastischen Beutel von der Flüssigkeit durchflossen werden muss. Infolge muss dieser bei einem Wechsel der Art der Flüssigkeit und nach jeder Nutzung gereinigt oder ausgetauscht werden, um eine Kontamination der Flüssigkeit durch Rückstände im Volumenstromsensor zu verhindern.

Um diesen Nachteil zu überwinden, gab es schon erste Bestrebungen Sensoren außen an den Beuteln anzubringen, welche zum Messen eines Flüssigkeitsniveaus im elastischen Beutel ausgebildet sind, wodurch bei Bekanntsein des Volumens des elastischen Beutels auf die Menge an Flüssigkeit im elastischen Beutel rückgerechnet werden kann. Eine Genauigkeit einer Anzeige des Flüssigkeitsniveaus hängt dabei aber von der Anzahl an Sensoren ab, die auf dem Beutel appliziert werden. Bei einer hohen Anzahl an Sensoren ist jedoch die Wahrscheinlichkeit groß, dass ein Ausfall einzelner Sensoren nicht sofort erkannt wird, wodurch das Flüssigkeitsniveau im elastischen Beutel nicht mehr korrekt ermittelt werden kann. Ferner ist so ein System aufgrund der hohen Anzahl an Sensoren teuer.
Es ist die Aufgabe der vorliegenden Erfindung ein System zur Messung einer Menge an Flüssigkeit in einem elastischen Beutel bereit zu stellen, das die Nachteile des Standes der Technik überwindet und bei dem mittels einer geringen Anzahl an Sensoren ohne direkten Kontakt mit der Flüssigkeit eine Menge an Flüssigkeit im elastischen Beutel feststellbar ist.

Erfindungsgemäß wird diese Aufgabenstellung durch ein System gemäß Anspruch 1 und ein Verfahren gemäß Anspruch 9 gelöst, und zwar dadurch, dass der elastische Beutel eine Gasöffnung aufweist, welche in die Gasphase reicht und dass der zumindest eine Sensor zum Messen eines Flüssigkeitsniveaus der Flüssigkeit im elastischen Beutel ausgebildet ist, wobei die Steuereinheit auf Basis des Flüssigkeitsniveaus und des Abstands zwischen den zumindest zwei Drückelementen zum Berechnen einer Menge an Flüssigkeit im elastischen Beutel ausgebildet ist und wobei durch eine Verringerung des Abstands zwischen den zumindest zwei Drückelementen mittels der zumindest einen Antriebseinheit das Flüssigkeitsniveau der Flüssigkeit im elastischen Beutel veränderbar ist.
Hierdurch ist der Vorteil erhalten, dass nur eine geringe Anzahl an Sensoren, genau genommen zumindest nur ein Sensor nötig ist, um mit ausreichender Genauigkeit das Flüssigkeitsniveau im elastischen Beutel zu ermitteln. Der zumindest eine Sensor ist vorteilhaft an einer bestimmten Höhe des elastischen Beutels entweder in einem Drückelement oder direkt an der Wandung des elastischen Beutels appliziert. Ist der zumindest ein Sensor zum Beispiel auf halber Höhe der Wandung des elastischen Beutels angebracht, schlägt dieser an, wenn das Flüssigkeitsniveau zur Hälfte abgefallen ist und der elastische Beutel halb leer ist. Durch die Steuereinheit wird dann der Abstand zwischen den zumindest zwei Drückelementen verringert, wodurch der elastische Beutel zusammengedrückt wird und wodurch das Flüssigkeitsniveau im elastischen Beutel wieder gehoben wird, obwohl sich eine geringere Menge an Flüssigkeit im elastischen Beutel befindet. Infolge wird aufgrund der erfindungsgemäßen Ausbildung des Systems die Messung der Menge an Flüssigkeit immer genauer, je weiter der Abstand zwischen den zumindest zwei Drückelementen verringert wird.

Zweckmäßig ist der zumindest eine Sensor durch einen kapazitiven Sensor oder einen optischen Sensor gebildet. Bei der Verwendung von zumindest einem kapazitiven Sensor sind dessen Sensorelemente vorteilhaft an gegenüberliegenden Wandungen des elastischen Beutels appliziert. In einer weiteren Ausführungsvariante können die Sensorelemente auch jeweils in den Drückelementen angeordnet sein.

Vorteilhaft sind bei Vorhandensein von mehreren Sensoren die Sensoren in gleichen Abständen zueinander entlang einer in Betriebslage im Wesentlichen vertikalen Achse an zumindest einem Drückelement angeordnet. Werden mehrere Sensoren eingesetzt, kann die Genauigkeit des Systems noch weiter erhöht werden, wobei das System bei einer gleichen Anzahl an Sensoren immer genauer arbeitet, als aus dem Stand der Technik bekannte Systeme.

Bevorzugt weist der elastische Beutel eine im Wesentlichen flächige Form auf. Besonders bevorzugt ist der elastische Beutel aus einer umgefalteten Kunststofffolie gebildet, welche an ihren Rändern zusammengeschweißt ist. Hierdurch ist der Vorteil erhalten, dass die Drückelemente, die vorteilhaft auch eine flächige Form aufweisen und bevorzugt durch Platten gebildet sind, flächig an den Wandungen des elastischen Beutels anliegen. Zweckmäßig ist eine Fläche der Platten, über die die Platten an den Wandungen des elastischen Beutels anliegen, gleich groß oder größer als Flächen der Wandungen. Bevorzugt sind die Platten parallel zueinander und parallel zum elastischen Beutel angeordnet. Hierdurch ist der Vorteil erhalten, dass bei einer Bewegung der Platten aufeinander zu der gesamte elastische Beutel zusammen gedrückt wird und Ausbeulungen im elastischen Beutel großteils vermieden werden können. Ferner ist der Vorteil erhalten, dass der elastische Beutel bis auf kleine Reste komplett entleert werden kann und somit eine unnötige Verschwendung von Flüssigkeit vermieden wird.

Vorteilhaft ist die Gasöffnung durch ein erstes Rohr gebildet, welches in Betriebslage des elastischen Beutels von einer oberen Seite des elastischen Beutels in die Gasphase ragt oder welches in Betriebslage des elastischen Beutels von einer unteren Seite des elastischen Beutels in die Gasphase ragt. Die Flüssigkeitsöffhung ist vorteilhaft durch ein zweites Rohr gebildet. Durch die Ausbildung der Gasöffnung und der Flüssigkeitsöffnung als Rohre können diese bei einem elastischen Beutel, gebildet durch eine umgefaltete an den Rändern verschweißte Kunststofffolie, sehr leicht bei der Produktion zwischen die umgefaltete Kunststofffolie gelegt werden und mitverschweißt werden. Ragt das erste Rohr von einer unteren Seite des elastischen Beutels in die Gasphase ist der Vorteil erhalten, dass die Gasöffnung und die Flüssigkeitsöffhung an einer Seite, nämlich in Betriebslage des elastischen Beutels an der unteren Seite des elastischen Beutels, angeordnet sind, wodurch die Handhabung des elastischen Beutels erleichtert wird.

Zweckmäßig weist das System eine Be- und Entlüftungseinheit auf, welche mit der Steuereinheit zum Kommunizieren verbunden ist und welche an die Gasöffnung des elastischen Beutels anschließt. Hierdurch ist der Vorteil erhalten, dass ein Flüssigkeitsdruck im elastischen Beutel mittels der Be- und Entlüftungseinheit gesteuert durch die Steuereinheit unabhängig von der Bewegung der Drückelemente regelbar ist.

Weitere vorteilhafte Ausführungsvarianten des erfindungsgemäßen Systems werden in weiterer Folge anhand der Figuren näher erläutert.
Figuren 1 und 2 zeigen eine erste Ausführungsvariante des erfindungsgemäßen Systems jeweils in einer schematischen Seitenansicht.
Figur 3 zeigt die erste Ausführungsvariante des erfindungsgemäßen Systems gemäß Figur 1 in einer schematischen Schnittansicht.
Figuren 4 und 5 zeigen jeweils eine weitere Ausführungsvariante des erfindungsgemäßen Systems in einer schematischen Schnittansicht.
Figur 6 zeigt eine weitere Ausführungsvariante des erfindungsgemäßen Systems bei einer Verwendung in einem Augenoperationsgerät in einer schematischen Darstellung.

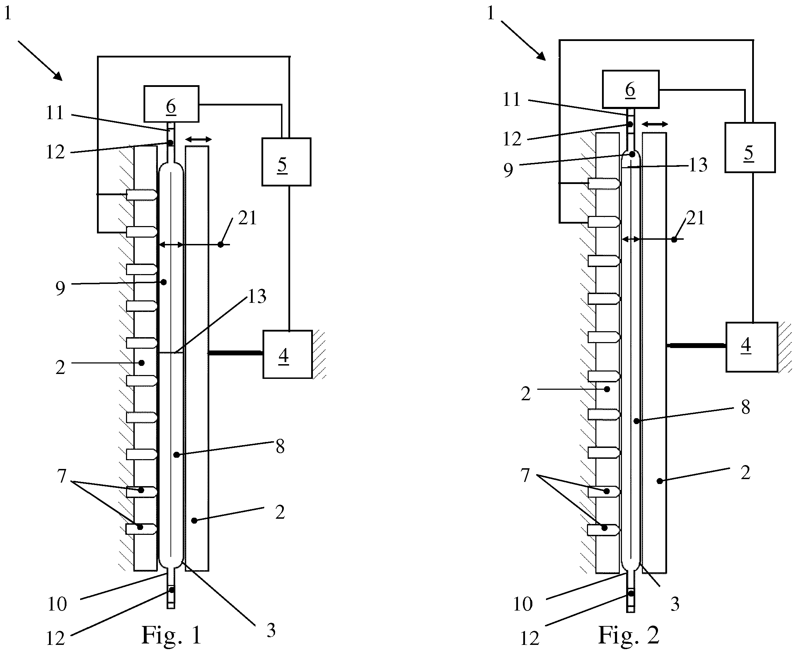

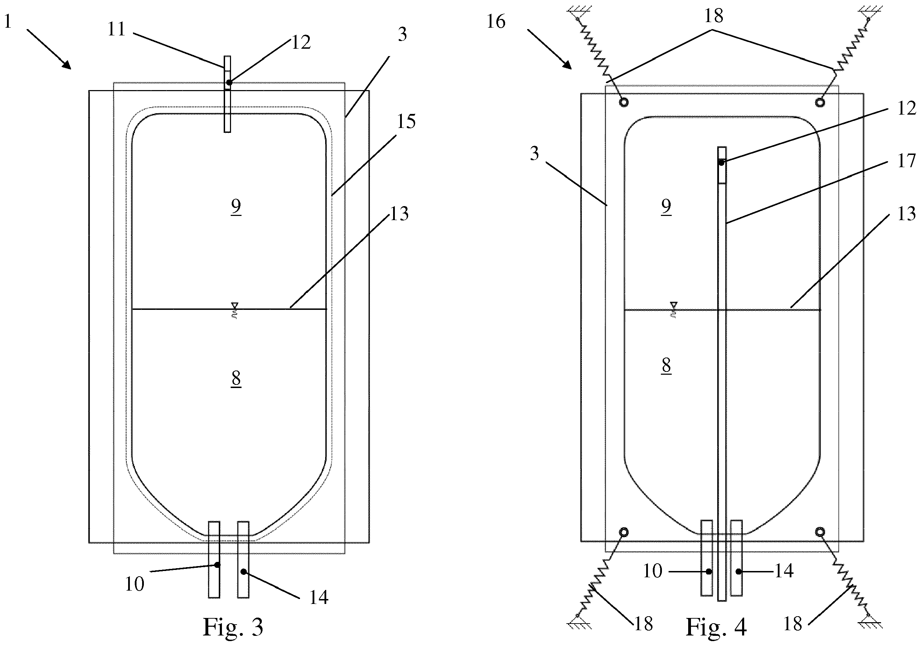

Figuren 1 und 2 zeigen eine erste Ausführungsvariante des erfindungsgemäßen Systems 1 jeweils in einer schematischen Seitenansicht. Das System 1 umfasst zwei durch Platten 2 gebildete Drückelemente, einen elastischen Beutel 3, eine Antriebseinheit 4, eine Steuereinheit 5, eine Be- und Entlüftungseinheit 6 und zehn Sensoren 7. Der elastische Beutel 3 weist eine Flüssigkeitsphase 8 und eine Gasphase 9 auf und ist in seiner Betriebslage vertikal ausgerichtet. Eine Flüssigkeit der Flüssigkeitsphase 8 kann zum Beispiel durch Infusionsflüssigkeit, insbesondere eine Kochsalzlösung, oder Irrigationsflüssigkeit gebildet sein. Ein Gas der Gasphase 9 ist vorteilhaft durch Luft gebildet. Der elastische Beutel 3 weist im unteren Bereich eine Injektionsöffnung und eine Flüssigkeitsöffnung auf, welche Flüssigkeitsöffnung durch ein zweites Rohr 10 gebildet ist und durch welche Flüssigkeit aus der Flüssigkeitsphase 8 des elastischen Beutels 3 abgegeben werden kann. Die Injektionsöffnung ist in Figur 3 dargestellt und dient zum Injizieren von Flüssigkeit oder Additiven, die zur Flüssigkeit im elastischen Beutel 3, zum Beispiel vor einem Operationsstart, zugegeben werden müssen. Die Injektionsöffnung ist durch ein drittes Rohr 14 gebildet. Im oberen Bereich des elastischen Beutels 3 ist eine Gasöffnung ausgebildet, welche Gasöffnung durch ein erstes Rohr 11 gebildet ist. Das erste Rohr 11 reicht in die Gasphase 9 des elastischen Beutels 3 und ist mit der Be- und Entlüftungseinheit 6 verbunden.

Die Platten 2 sind parallel und beabstandet zueinander angeordnet und liegen jeweils an gegenüberliegenden Wandungen des elastischen Beutels 3 an. Infolge ist der elastische Beutel 3 zwischen den Platten 2 eingeklemmt angeordnet. Eine Platte 2 ist fixiert und die andere Platte 2 ist beweglich gelagert, wobei die beweglich gelagerte Platte 2 so mittels der Antriebseinheit 4 antreibbar ist, dass die Platten 2 aufeinander zu bewegbar sind, wodurch sich ein Abstand 21 zwischen den Platten 2 verkleinern lässt. Vorteilhaft ist die Antriebseinheit 4 durch einen mit einem Elektromotor angetriebenen Zahnstangenantrieb, einen mit einem Elektromotor angetriebenen Gewindestangenantrieb, einen pneumatisch angetriebenen Zylinder oder einen hydraulisch angetriebenen Zylinder gebildet.

Sowohl die Sensoren 7, also auch die Be- und Entlüftungseinheit 6 und die Antriebseinheit 4 sind mit der Steuereinheit 5 zum Kommunizieren verbunden. Der besseren Übersichtlichkeit halber sind in Figuren 1 und 2 nicht sämtliche zehn Sensoren 7 mit der Steuereinheit 5 verbunden. Die Sensoren 7 sind durch optische Sensoren gebildet, wobei diese dann ein Sensorsignal abgeben, das die Detektion von Flüssigkeit kennzeichnet, wenn deren Messfühlerspitze im Bereich der Flüssigkeitsphase 8 an dem Beutel 3 anliegen. Derartige optische Sensoren sind dem Fachmann bekannt. Die Sensoren 7 sind in gleichen Abständen zueinander entlang einer in Betriebslage des Systems 1 vertikalen Achse an einer Platte 2 angeordnet. Infolge kann durch die Steuereinheit 5 mittels der Sensoren 7 ein Flüssigkeitsniveau 13 im elastischen Beutel 3 detektiert werden.

Die Be- und Entlüftungseinheit 6 umfasst ein Proportionaldruckventil und eine Kompressoreinheit zum Komprimieren von Umgebungsluft.

Sowohl in dem ersten Rohr 11, als auch im zweiten Rohr 10 und im dritten Rohr 14 sind jeweils Brechsicherungen 12 ausgebildet, wobei die Rohre 10, 11 und 14 erst für den Betrieb bei einer Operation freigegeben sind, wenn die Brechsicherungen 12 geknickt wurden.

Im Folgenden wird die Funktionsweise des erfindungsgemäßen Systems 1 näher beschrieben, wobei von einem mit der Flüssigkeit voll gefüllten elastischen Beutel 3 ausgegangen wird. Dazu wurde entweder ein neu befüllter elastischer Beutel 3 zwischen den Platten 2 angeordnet oder es wird ein bereits zwischen den Platten 2 angeordneter elastischer Beutel 3 mittels eines Zusatzgeräts über das dritte Rohr 14 mit der Flüssigkeit befüllt.

Mittels des Proportionaldruckventils der Be- und Entlüftungseinheit 6 wird gesteuert durch die Steuereinheit 5 durch eine Gaszufuhr in den elastischen Beutel 3 und/ oder eine Gasabfuhr aus dem elastischen Beutel 3 ein Druck im elastischen Beutel 3 geregelt. Der Druck im elastischen Beutel 3 wird dabei vorteilhaft direkt an der Steuereinheit 5 eingestellt. Der Flüssigkeitsdruck in dem zweiten Rohr 10 ist durch das Gewicht der Flüssigkeit geringfügig höher als der durch das Proportionaldruckventil im elastischen Beutel 3 eingestellte Druck, wobei zur Vermeidung von unerwünschtem Tropfen aus dem zweiten Rohr 10 kurzfristig mittels der Be- und Entlüftungseinheit 6 der elastische Beutel 3 mit einem Druck geringer als ein Umgebungsdruck beaufschlagt werden kann. Bei Vorsehen eines zusätzlichen Ventils, angeschlossen an das zweite Rohr 10, kann diese Funktion entfallen. Bei dem System 1 wird die Abgabe von Flüssigkeit nur durch die Be- und Entlüftungseinheit 6 gesteuert, wobei schon ein geringfügig höherer Druck als der Umgebungsdruck oder ein Druck gleich dem Umgebungsdruck, je nach Menge an Flüssigkeit im elastischen Beutel 3, ausreicht, um Flüssigkeit aus dem elastischen Beutel 3 abzugeben. Die Sensoren 7 erfassen während der Abgabe von Flüssigkeit aus dem zweiten Rohr 10 kontinuierlich das Flüssigkeitsniveau 13, wobei bei Unterschreiten eines vorher vorgegebenen Flüssigkeitsniveaus 13 die Steuereinheit 5 die Antriebseinheit 4 ansteuert, die Platten 2 aufeinander zu zu bewegen, um den Abstand 21 zwischen den Platten 2 zu verringern. Durch das aufeinander zu Bewegen der Platten 2 wird ein Volumen des elastischen Beutels 3 verringert und in weiterer Folge ein Volumen der Gasphase 9 verkleinert.

Bei dem in Figur 1 dargestellten erfindungsgemäßen System 1 ist das Flüssigkeitsniveau 13 bis zur Hälfte abgesunken und der elastische Beutel 3 ist nur mehr halb gefüllt. Bei dem in Figur 2 dargestellten erfindungsgemäßen System 1 wurde der Abstand 21 zwischen den Platten 2 gegenüber dem Abstand 21 gemäß Figur 1 um die Hälfte verringert, wodurch das Flüssigkeitsniveau 13 wieder bis oben hin im elastischen Beutel 3 angestiegen ist. Sinkt das Flüssigkeitsniveau 13 in weiterer Folge wieder bis zur Hälfte ab, ist der elastische Beutel 3 nur noch mit einem Viertel der ursprünglichen Menge an Flüssigkeit befüllt. Dieser Vorgang wird solange fortgesetzt, bis der elastische Beutel 3 leer ist, oder bis der elastische Beutel 3 bis zu einer vorgegebenen Menge entleert worden ist.

Die Steuereinheit 5 ist durch Kenntnis des Abstands 21 zwischen den Platten 2 und der durch die Sensoren 7 gemessenen Lage des Flüssigkeitsniveaus 13 dazu ausgebildet die Menge an im elastischen Beutel 3 vorhandenen Flüssigkeit zu erfassen, wobei eine Messgenauigkeit der Menge an vorhandener Flüssigkeit bei Verringerung des Abstands 21 zunimmt. Durch die Bewegung der Platten 2 aufeinander zu und die daraus resultierende Verringerung des Abstands 21 zwischen den Platten 2 folgt somit nicht nur eine Verringerung des Volumens der Gasphase 9, wodurch eine Trägheit des Systems 1 bei Druckveränderung vermieden wird, sondern es wird auch die Messgenauigkeit der Menge an Flüssigkeit, die im elastischen Beutel 3 noch vorhanden ist, bei gleichbleibender Anzahl an Sensoren 7 erhöht.

In einer weiteren Ausführungsvariante sind die Sensoren 7 durch kapazitive Sensoren gebildet, wobei jeweils ein kapazitiver Sensor zwei Sensorelemente aufweist, die gegenüberliegend jeweils an den Platten 2 befestigt sind.

Figur 3 zeigt die erste Ausführungsvariante des erfindungsgemäßen Systems 1 nach Figur 1 in einer schematischen Schnittansicht. Der elastische Beutel 3 besteht aus einer einstückigen Kunststofffolie, die umgefaltet ist und an Rändern 15 der Kunststofffolie verschweißt ist.

Figur 4 zeigt eine weitere Ausführungsvariante des erfindungsgemäßen Systems 16 in einer schematischen Frontansicht. Das System 16 unterscheidet sich zu dem in den Figuren 1 bis 3 gezeigten System 1 dadurch, dass es Auseinanderziehelemente in Form von Federn 18 aufweist. Mittels der Federn 18 wird der elastische Beutel 3 quer zu der Bewegung der beweglich gelagerten Platten 2 beim aufeinander zu Bewegen der Platten 2 auseinander gezogen, wodurch ein Zusammenfallen bzw. ein Zusammenfalten des elastischen Beutels 3 verhindert wird und dieser im Wesentlichen seine Form behält.

Ferner reicht bei dem System 16 ein die Gasöffnung bildendes erstes Rohr 17 von einer unteren Seite des elastischen Beutels 3 in die Gasphase 9. Hierdurch ist der Vorteil erhalten, dass sämtliche Anschlüsse auf einer Seite des elastischen Beutels 3 angeordnet sind.

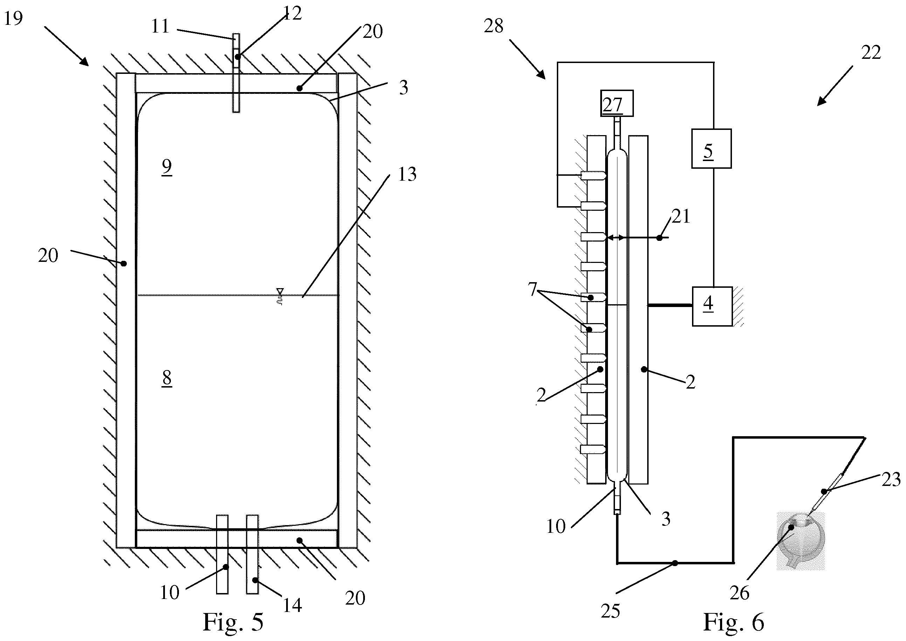

Figur 5 zeigt eine weitere Ausführungsvariante des erfindungsgemäßen Systems 19 in einer schematischen Frontansicht. Das System 19 unterscheidet sich zu dem in Figur 1 gezeigten System 1 dadurch, dass das System 19 Seitenwände 20 aufweist. Die Seitenwände 20 sind jeweils an Zusatzelementen, insbesondere einem Rahmen, fixiert, und sind durch weitere Platten gebildet, wobei die angetriebene Platte 2 sich relativ zu den weiteren Platten bewegen kann. Hierdurch ist der Vorteil erhalten, dass ein seitliches Ausweichen des elastischen Beutels 3 oder ein Ausbeulen des elastischen Beutels 3 auch bei hohen Drücken vermieden wird, wodurch der Flüssigkeitsdruck auch bei hohen Drücken sehr gut regelbar ist und es zu keiner Trägheit des Systems 19 aufgrund der Ausdehnung des elastischen Beutels 3 kommen kann.

Figur 6 zeigt eine weitere Ausführungsvariante des erfindungsgemäßen Systems 28 bei einer Verwendung in einem Augenoperationsgerät 22 in einer schematischen Darstellung. Das System 28 unterscheidet sich dadurch zu dem in den Figuren 1 bis 3 gezeigten System 1, dass bei dem System 28 die Be- und Entlüftungseinheit durch einen einfachen Filter 27 gebildet ist.

Das Augeoperationsgerät 22 umfasst zusätzlich zum System 28 ein chirurgisches Handstück 23, wobei das chirurgische Handstück 23 mittels eines Schlauchs 25 direkt an das zweite Rohr 10 angeschlossen ist und ein nicht dargestelltes Regelventil aufweist. Das Regelventil kann zum Beispiel durch ein Magnetventil gebildet sein, wobei über das Regelventil eine Menge an Flüssigkeit, das in ein Auge 26 abgegeben wird, geregelt wird. Bei der Abgabe an Flüssigkeit in das Auge 26 strömt über den Filter 27 Umgebungsluft in den elastischen Beutel 3 nach und bei einer Verringerung des Abstands 21 wird Luft aus dem elastischen Beutel 3 über den Filter 27 in die Umgebung abgelassen. Ein für eine Operation nötiger Irrigationsdruck wird durch einen Höhenunterschied zwischen dem elastischen Beutel 3 und dem chirurgischen Handstück 23 erreicht. Über die Sensoren 7 und den Abstand 21 zwischen den Platten 2 wird von der Steuereinheit 5 kontinuierlich die Menge an Flüssigkeit im elastischen Beutel 3 detektiert und bei Unterschreiten einer bestimmten Menge an Flüssigkeit im elastischen Beutel 3 wird von der Steuereinheit 5 ein Wahnsignal ausgegeben.

In einer weiteren Ausführungsvariante ist die Be- und Entlüftungseinheit 6 durch ein durch die Steuereinheit 5 ansteuerbares Ventil gebildet.

Es kann erwähnt werden, dass eine erfindungsgemäße Vorrichtung zur Messung der Menge an Flüssigkeit als Füllstandsanzeiger bei einer Vielzahl an Geräten der unterschiedlichsten Fachgebiete verwendet werden kann. So ein Füllstandsanzeiger könnte beispielsweise zur Messung des Füllstands der Scheibenwischerflüssigkeit in einem Auto oder zur Messung des Füllstandes eines Saftgetränks in einem Getränkeautomaten verwendet werden. Bei einem Melkautomaten könnte die Menge der bereits gemolkenen Milch und bei einem Harnbeutel die von einem Patienten bereits abgegebene Harnmenge gemessen werden.

Die Steuereinrichtung könnte auch Sensorsignale von in unterschiedlichen Abständen zueinander angebrachten Sensoren verarbeiten, wobei die Auflösung der Messung durch näher beieinander liegende Sensoren für einen bestimmten Flüssigkeitsspiegel noch erhöht werden könnte. So könnten beispielsweise nur in mittlerer Höhe des Beutels eine Vielzahl an Sensoren nahe beieinander angebraucht sein, wobei die Stellung der Drückelemente dafür sorgt, dass der Flüssigkeitsspiegel immer im Messbereich dieser Sensoren gehalten wird. Hierdurch ist der Vorteil erhalten, dass mit weniger Sensoren eine sehr hohe Auflösung der Messung erreicht werden kann.

## Patentansprüche

1. System (1, 16, 19, 28) zur Messung einer Menge an Flüssigkeit in einem elastischen Beutel (3), wobei der elastische Beutel (3) zusätzlich zur durch die Flüssigkeit gebildeten Flüssigkeitsphase (8) eine Gasphase (9) aufweist und wobei in Betriebslage des elastischen Beutels (3) im unteren Bereich des elastischen Beutels (3) eine Flüssigkeitsöffnung (10) zur Entnahme von Flüssigkeit aus dem elastischen Beutel (3) ausgebildet ist,
mit zumindest zwei Drückelementen, welche an gegenüberliegenden Wandungen des elastischen Beutels (3) anliegen und zueinander beabstandet sind,
zumindest einer Antriebseinheit (4), welche zum Antreiben zumindest eines Drückelements ausgebildet ist, wobei die zumindest zwei Drückelemente durch Antreiben zumindest eines Drückelements aufeinander zu bewegbar sind,
zumindest einem Sensor (7), und
einer Steuereinheit (5), welche zum Kommunizieren mit dem zumindest einen Sensor (7) und mit der zumindest einen Antriebseinheit (4) ausgebildet ist,
**dadurch gekennzeichnet, dass** der elastische Beutel (3) eine Gasöffnung aufweist, welche in die Gasphase (9) reicht und dass der zumindest eine Sensor (7) zum Messen eines Flüssigkeitsniveaus (13) der Flüssigkeit im elastischen Beutel (3) ausgebildet ist, wobei die Steuereinheit (5) auf Basis des Flüssigkeitsniveaus (13) und des Abstands (21) zwischen den zumindest zwei Drückelementen zum Berechnen einer Menge an Flüssigkeit im elastischen Beutel (3) ausgebildet ist und wobei durch eine Verringerung des Abstands (21) zwischen den zumindest zwei Drückelementen mittels der zumindest einen Antriebseinheit (4) das Flüssigkeitsniveau (13) der Flüssigkeit im elastischen Beutel (3) veränderbar ist.

2. System (1, 16, 19, 28) nach Anspruch 1, **dadurch gekennzeichnet, dass** der zumindest eine Sensor (7) durch einen kapazitiven Sensor oder einen optischen Sensor gebildet ist.

3. System (1, 16, 19, 28) nach Anspruch 1 oder 2, **dadurch gekennzeichnet, dass** bei Vorhandsein von mehreren Sensoren (7) die Sensoren (7) in gleichen Abständen zueinander entlang einer in Betriebslage des Systems (1, 16, 19, 28) im Wesentlichen vertikalen Achse an zumindest einem Drückelement angeordnet oder am elastischen Beutel 3 appliziert sind.

4. System (1, 16, 19, 28) nach einem der Ansprüche 1 bis 3, **dadurch gekennzeichnet, dass** der elastische Beutel (3) eine im Wesentlichen flächige Form aufweist.

5. System (1, 16, 19, 28) nach einem der Ansprüche 1 bis 4, **dadurch gekennzeichnet, dass** der elastische Beutel (3) aus einer umgefalteten Kunststofffolie gebildet ist, welche an ihren Rändern (15) zusammengeschweißt ist.

6. System (1, 16, 19, 28) nach einem der Ansprüche 1 bis 5, **dadurch gekennzeichnet, dass** die Gasöffnung durch ein erstes Rohr (11, 17) gebildet ist, welches in Betriebslage des elastischen Beutels (3) von einer oberen Seite des elastischen Beutels (3) in die Gasphase (9) ragt oder welches in Betriebslage des elastischen Beutels (3) von einer unteren Seite des elastischen Beutels (3) in die Gasphase (9) ragt.

7. System (1, 16, 19, 28) nach einem der Ansprüche 1 bis 6, **dadurch gekennzeichnet, dass** die zumindest zwei Drückelemente durch Platten (2) gebildet sind, die parallel zueinander angeordnet sind.

8. System (1, 16, 19) nach einem der Ansprüche 1 bis 7, **dadurch gekennzeichnet, dass** das System (1, 16, 19) eine Be- und Entlüftungseinheit (6) aufweist, welche mit der Steuereinheit (5) zum Kommunizieren verbunden ist und welche an die Gasöffnung des elastischen Beutels (3) anschließt.

9. Verfahren zur Messung einer Menge an Flüssigkeit in einem elastischen Beutel (3), welcher eine Gasphase (9) aufweist, mit einem System (1, 16, 19, 28) gemäß einem der Ansprüche 1 bis 8, **dadurch gekennzeichnet, dass** bei Unterschreiten eines festgelegten Flüssigkeitsniveaus (13) im elastischen Beutel (3) die zumindest eine Antriebseinheit (4) angesteuert wird den Abstand (21) zwischen den zumindest zwei Drückelemente zu verringern.

## Claims

1. A system (1, 16, 19, 28) for measuring an amount of liquid in an elastic bag (3), wherein the elastic bag (3) has a gas phase in addition to the liquid phase (8) formed by the liquid and wherein in the operational position of the elastic bag (3) there is formed in the lower area of the elastic bag (3) a liquid opening (10) for retrieving liquid from the elastic bag (3),
having at least two push elements, which abut at opposite walls of the elastic bag (3) and which are spaced apart from one another,
at least one drive unit (4), which is configured to drive at least one push element, wherein the at least two push elements may be moved towards one another by driving at least one push element,
at least one sensor (7), and
a control unit (5), which is configured to communicate with the at least one sensor (7) and the at least one drive unit (4),
**characterized in that** the elastic bag (3) has a gas opening, which extends into the gas phase (9), and that the at least one sensor (7) is configured to measure a liquid level (13) of the liquid in the elastic bag (3), wherein the control unit (5) is configured to calculate an amount of liquid in the elastic bag (3) on the basis of the liquid level (13) and the distance between the at least two push elements and wherein the liquid level (13) of the liquid in the elastic bag (3) may be changed by reducing the distance (21) between the at least two push elements by means of the at least one drive unit (4).

2. A system (1, 16, 19, 28) according to claim 1, **characterized in that** the at least one sensor (7) is formed by a capacitive sensor or an optical sensor.

3. A system (1, 16, 19, 28) according to claim 1 or 2, **characterized in that**, if several sensors (7) are present, the sensors (7) are arranged on at least one push element or applied onto the elastic bag (3) in equal intervals to one another along an in the operational position of the system (1, 16, 19, 28) essentially vertical axis.

4. A system (1, 16, 19, 28) according to any of claims 1 to 3, **characterized in that** the elastic bag (3) has an essentially plane shape.

5. A system (1, 16, 19, 28) according to any of claims 1 to 4, **characterized in that** the elastic bag (3) is formed from a plastic film that is folded over, which is sealed at the edges (15) thereof.

6. A system (1, 16, 19, 28) according to any of claims 1 to 5, **characterized in that** the gas opening is formed by a first tube (11, 17), which in the operational position of the elastic bag (3) projects from an upper side of the elastic bag (3) into the gas phase (9) or which in the operational position of the elastic bag (3) projects from a lower side of the elastic bag (3) into the gas phase (9).

7. A system (1, 16, 19, 28) according to any of claims 1 to 6, **characterized in that** the at least two push elements are formed by plates (2), which are arranged in parallel to one another.

8. A system (1, 16, 19) according to any of claims 1 to 7, **characterized in that** the system (1, 16, 19) has a ventilation unit (6), which is connected to the control unit (5) for communication and which connects to the gas opening of the elastic bag (3).

9. A method for measuring an amount of liquid in an elastic bag (3), which has a gas phase (9), having a system (1, 16, 19, 28) according to any of claims 1 to 8, **characterized in that**, if it dips below a determined liquid level (13) in the elastic bag (3), the at least one drive unit (4) is controlled to reduce the distance (21) between the at least two push elements.

## Revendications

1. Système (1, 16, 19, 28) pour mesurer une quantité de liquide dans un sachet élastique (3), le sachet élastique (3) contenant, en supplément à la phase liquide (8) formée par le liquide, une phase gazeuse (9), et en configuration de fonctionnement du sachet élastique (3) une ouverture pour liquide (10) est ménagée dans la zone inférieure du sachet élastique (3) pour prélever du liquide hors du sachet élastique (3),
comportant
au moins deux éléments presseurs qui s'appuient contre des parois opposées du sachet élastique (3) et qui sont espacés l'un de l'autre,
au moins une unité d'entraînement (4) qui est réalisée pour entraîner au moins un élément presseur, lesdits au moins deux éléments presseurs étant mobiles l'un vers l'autre par entraînement d'au moins un élément presseur,
au moins un capteur (7), et
une unité de commande (5) qui est réalisée pour communiquer avec ledit au moins un capteur (7) et avec ladite au moins une unité d'entraînement (4), **caractérisé en ce que**
le sachet élastique (3) présente une ouverture pour gaz qui va jusque dans la phase gazeuse (9), et **en ce que** ledit au moins un capteur (7) est réalisé pour mesurer un niveau de liquide (13) du liquide dans le sachet élastique (3), l'unité de commande (5) étant basée sur le niveau de liquide (13) et de la distance (21) entre lesdits au moins deux éléments presseurs pour calculer une quantité de liquide dans le sachet élastique (3) et, en réduisant la distance (21) entre lesdits au moins deux éléments presseurs, le niveau de liquide (13) du liquide dans le sachet élastique (3) est variable au moyen de ladite au moins une unité d'entraînement (4).

2. Système (1, 16, 19, 28) selon la revendication 1, **caractérisé en ce que** ledit au moins un capteur (7) est formé par un capteur capacitif ou par un capteur optique.

3. Système (1, 16, 19, 28) selon la revendication 1 ou 2, **caractérisé en ce qu'**en présence de plusieurs capteurs (7), les capteurs (7) sont agencés sur au moins un élément presseur de façon équidistante le long d'un axe sensiblement vertical en configuration de fonctionnement du système (1, 16, 19, 28) ou bien ils sont appliqués contre le sachet élastique (3).

4. Système (1, 16, 19, 28) selon l'une des revendications 1 à 3, **caractérisé en ce que** le sachet élastique (3) présente une forme sensiblement plane.

5. Système (1, 16, 19, 28) selon l'une des revendications 1 à 4, **caractérisé en ce que** le sachet élastique (3) est formé par une feuille pliée en matière plastique qui est soudée au niveau de ses bords (15).

6. Système (1, 16, 19, 28) selon l'une des revendications 1 à 5, **caractérisé en ce que** l'ouverture pour gaz est formée par un premier tube (11, 17) qui, en configuration de fonctionnement du sachet élastique (3), pénètre dans la phase gazeuse (9) depuis un côté supérieur du sachet élastique (3) ou qui, en configuration de fonctionnement du sachet élastique (3), pénètre dans la phase gazeuse (9) depuis un côté inférieur du sachet élastique (3).

7. Système (1, 16, 19, 28) selon l'une des revendications 1 à 6, **caractérisé en ce que** lesdits au moins deux éléments presseurs sont formés par des plaques (2) qui sont agencées parallèlement l'une à l'autre.

8. Système (1, 16, 19, 28) selon l'une des revendications 1 à 7, **caractérisé en ce que** le système (1, 16, 19) présente une unité d'aération et de ventilation (6) qui est reliée à l'unité de commande (5) pour la communication et qui se raccorde à l'ouverture pour gaz du sachet élastique (3).

9. Procédé pour mesurer une quantité de liquide dans un sachet élastique (3) qui présente une phase gazeuse (9) au moyen d'un système (1, 16, 19, 28) selon l'une des revendications 1 à 8, **caractérisé en ce que** lorsqu'on passe au-dessous d'un niveau de liquide fixé (13) dans le sachet élastique (3), ladite au moins une unité d'entraînement (4) est pilotée pour réduire la distance (21) entre lesdits au moins deux éléments presseurs.
